# EUROPEAN PATENT APPLICATION

(11) **EP 2 005 979 A2**
(43) Date of publication of application: **24.12.2008**
(21) Application number: 08158640.6
(22) Date of filing: 19.06.2008
(51) Int. Cl.: A61L 31/04, A61L 31/14

(54) **Multiple point detacher system**

(30) Priority: 22.06.2007 US 767258
(71) Applicant: Neurovasx, Inc., Maple Grove, MN 55369 (US)
(72) Inventor: Calabria, Marie F, Plymouth, MN 55446 (US); Bertelson, Arthur J, Buffalo, MN 55313 (US)
(74) Representative: Curley, Donnacha John

(57) **Abstract**

Embodiments of the invention include a method for treating an aneurysm, comprising: providing a biocompatible polymeric sleeve, string or coil or combination of sleeve, string or coil, made from a material selected from one or more of a group consisting of an acrylamide, a methacrylate, cyclodextran, synthetic elastin polymer, poly chelating amphiphilic polymers, hydrogels, hyaluronic acid conjugates, polyanhydrides, glycolipids, polysaccharides, and halamines, natural hydrogel, a synthetic hydrogel, silicone, polyurethane, polysulfone, cellulose, polyethylene, polypropylene, polyamide, polyimide, polyester, polytetrafluoroethylene, polyvinyl chloride, epoxy, phenolic, neoprene, polyisoprene, and a combination thereof.; transporting the sleeve, string or coil to an aneurysm; filling the aneurysm with the sleeve, coil, or string; and detaching the sleeve, string or coil

## Description

### CLAIM OF PRIORITY

This Continuation-in-Part application claims priority to U.S. Patent Application Serial No. 10/967,667, which claims priority from U.S. Patent Application Serial No. 60/481,762, filed December 10, 2003. These applications are incorporated herein by reference.

The inventive subject matter described herein relates to an aneurysm embolization material embodiments and to method embodiments for repairing an aneurysm. The inventive subject matter also relates to method embodiments for making, a method for using and to method embodiments for detaching an aneurysm filler detacher wire.

### COPYRIGHT

A portion of the disclosure of this patent document contains material that is subject to copyright protection. The copyright owner has no objection to the facsimile reproduction by anyone of the patent document or the patent disclosure, as it appears in the Patent and Trademark Office patent files or records, but otherwise reserves all copyright rights whatsoever. The following notice applies to the products, processes and data as described below and in the tables that form a part of this document: Copyright 2007, Neurovasx, Inc. All Rights Reserved.

### BACKGROUND OF THE INVENTION

An aneurysm is a balloon-like swelling in a wall of a blood vessel. Aneurysms result in weakness of the vessel wall in which it occurs. This weakness predisposes the vessel to tear or rupture with potentially catastrophic consequences for any individual having the aneurysm. Vascular aneurysms are a result of an abnormal dilation of a blood vessel, usually resulting from disease and/or genetic predisposition which can weaken the arterial wall and allow it to expand. Aneurysm sites tend to be areas of mechanical stress concentration so that fluid flow seems to be the most likely initiating cause for the formation of these aneurysms.

Aneurysms in cerebral circulation tend to occur in an anterior communicating artery, posterior communicating artery, and a middle cerebral artery. The majority of these aneurysms arise from either curvature in the vessels or at bifurcations of these vessels. The majority of cerebral aneurysms occur in women. Cerebral aneurysms are most often diagnosed by the rupture and subarachnoid bleeding of the aneurysm.

Cerebral aneurysms are most commonly treated in open surgical procedures where the diseased vessel segment is clipped across the base of the aneurysm. While considered to be an effective surgical technique, particularly considering an alternative which may be a ruptured or re-bleed of a cerebral aneurysm, conventional neurosurgery suffers from a number of disadvantages. The surgical procedure is complex and requires experienced surgeons and well-equipped surgical facilities. Surgical cerebral aneurysm repair has a relatively high mortality and morbidity rate of about 2% to 10%.

Current treatment options for cerebral aneurysm fall into two categories, surgical and interventional. The surgical option has been the long held standard of care for the treatment of aneurysms. Surgical treatment involves a long, delicate operative procedure that has a significant risk and a long period of postoperative rehabilitation and critical care. Successful surgery allows for an endothelial cell to endothelial cell closure of the aneurysm and therefore a cure for the disease. If an aneurysm is present within an artery in the brain and bursts, this creates a subarachnoid hemorrhage, and a possibility that death may occur. Additionally, even with successful surgery, recovery takes several weeks and often requires a lengthy hospital stay.

In order to overcome some of these drawbacks, interventional methods and prostheses have been developed to provide an artificial structural support to the vessel region impacted by the aneurysm. The structural support must have an ability to maintain its integrity under blood pressure conditions and impact pressure within an aneurysmal sac and thus prevent or minimize a chance of rupture. U.S. Patent No. 5,405,379 to Lane, discloses a self-expanding cylindrical tube which is intended to span an aneurysm and result in isolating the aneurysm from blood flow. While this type of stent-like device may reduce the risk of aneurysm rupture, the device does not promote healing within the aneurysm. Furthermore, the stent may increase a risk of thrombosis and embolism. Additionally, the wall thickness of the stent may undesirably reduce the fluid flow rate in a blood vessel. Stents typically are not used to treat aneurysms in a bend in an artery or in tortuous vessels such as in the brain because stents tend to straighten the vessel.

Patent No.5,354,295 to Guglielmi et al., describes a type ofvasoclusion coil. Disadvantages of use of this type of coil are that the coil may compact, may migrate over time, and the coil does not optimize the patient's natural healing processes.

### DESCRIPTION OF THE DRAWINGS

FIG. 1 is a side view of one embodiment of a catheter used for repairing an aneurysm with the method of the present invention.
FIG. 2 is a schematic view of one embodiment of delivery of a hydrogel sleeve, coil or string to an aneurysm sac.
FIG. 3 is a radial cross-sectional view of one embodiment of the hydrogel sleeve, coil or string of the present invention.
FIG. 4 is a side view of a distal tip of a catheter used on the method of the present invention, the tip comprising a mechanism for heating the hydrogel string to terminate the string.
FIG. 5A is a side view of one mechanical cutter mechanism for cutting the hydrogel string.
FIG. 5B is a side view of the mechanical cutter mechanism of FIG. 5a in a closed position.
FIG. 6 is a side view of one hollow sleeve, coil, or string embodiment of the present invention positioned proximal to an aneurysm sac.
FIG. 7 is a side view of the hollow sleeve, coil, or string embodiment of FIG. 6 wherein the hollow sleeve, coil, or string is positioned within the aneurysm sac.
FIG. 8A illustrates a side view of a detachable filler material section with cut portions.
FIG. 8B illustrates another embodiment of the detachable filler material section with cut portions.
FIG. 9A illustrates a side view of one embodiment of a coil loop and coil loop straightening sheath, with the loop coiled and exposed.
FIG. 9B illustrates the coil loop and coil loop straightening sheath of FIG. 9A wherein the coil loop is partially straightened.
FIG. 9C illustrates the coil loop and coil loop straightening sheath of FIG. 9A wherein the coil loop is fully straightened.
FIG. 10 illustrates a side view of one embodiment of a coiled sleeve, coil, or string.
FIG. 11 illustrates a perspective view of one aneurysm filler detacher wire of the invention.
FIG. 12 is a side view of a heater element of the catheter device of the invention.
FIG. 13 is a side view of a distal tip of a catheter used with one method embodiment of the invention, the tip comprising a mechanism for heating the hydrogel string to terminate the string.
FIGs. 14A and 14C illustrate side views of one mechanical cutter mechanism for cutting the hydrogel string.
FIGs. 14B and 14D illustrate side views of the mechanical cutter mechanism of FIG. 5A in a closed position.
FIG. 15A illustrates a perspective view of one feedback mechanism for detachment of a string, coil or sleeve from a catheter or micro catheter.
FIG. 15B illustrates a perspective view of one other feedback mechanism for detachment of a string, coil or sleeve from a catheter or microcatheter.
FIG. 16 illustrates another embodiment of a feedback mechanism.
FIG. 17 illustrates one embodiment of a hollow tubular aneurysm filler.
FIG. 18 illustrates another embodiment of a hollow tubular aneurysm filler.
FIG. 19 illustrates one embodiment of a microcatheter used in a method of the invention.
FIG. 20 illustrates one perspective view of filler that includes a fabric.
Fig 21 illustrates a perspective view of another filler that includes a fabric.
FIGs. 22A and 22B and 22C illustrate cross-sectional views of filler embodiments that include a fabric.
FIG. 23 illustrates a side view of a solid filler embodiment.
FIG. 24 illustrates a side view of a ribbon filler embodiment.
FIG. 25 illustrates a side view of a braided filler embodiment.
FIG. 26 illustrates one embodiment of a flexible distal tip in a first position.
FIG. 27 illustrates the embodiment of FIG. 26 in an expanded position.
FIG. 28 illustrates one embodiment for a ribbon marker for a detacher joint.
FIG. 29 illustrates another embodiment for a ribbon marker for a detacher joint.
FIG. 30 illustrates a cross-sectional view of a system that includes an aneurysm filler, a detacher and a delivery mechanism.
FIG. 31 illustrates a cross-sectional view of a detacher system that includes the system of FIG. 30.
FIG. 32 illustrates a cross-sectional view of a detacher system that includes the system of FIG. 31.
FIG. 33 illustrates a cross-sectional view of another detacher system
FIG. 34 illustrates a cross-sectional view of another detacher system.

### DETAILED DESCRIPTION

Although detailed embodiments of the invention are disclosed herein, it is to be understood that the disclosed embodiments are merely exemplary of the invention that may be embodied in various and alternative forms. Specific structural and functional details disclosed herein are not to be interpreted as limiting, but merely as a basis for teaching one skilled in the art to variously employ the aneurysm filler detacher wire embodiments. Throughout the drawings, like elements are given like numerals.

Referred to herein are trade names for materials including, but not limited to, polymers and optional components. The inventors herein do not intend to be limited by materials described and referenced by a certain trade name. Equivalent materials (e.g., those obtained from a different source under a different name or catalog (reference) number to those referenced by trade name may be substituted and utilized in the methods described and claimed herein. All percentages and ratios are calculated by weight unless otherwise indicated. All percentages are calculated based on the total composition unless otherwise indicated. All component or composition levels are in reference to the active level of that component or composition, and are exclusive of impurities, for example, residual solvents or by-products, which may be present in commercially available sources.

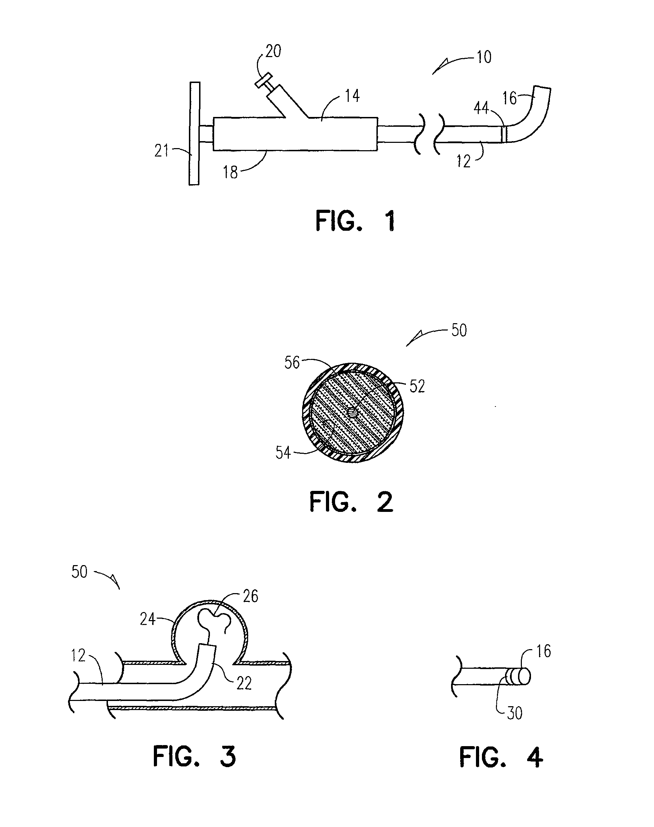

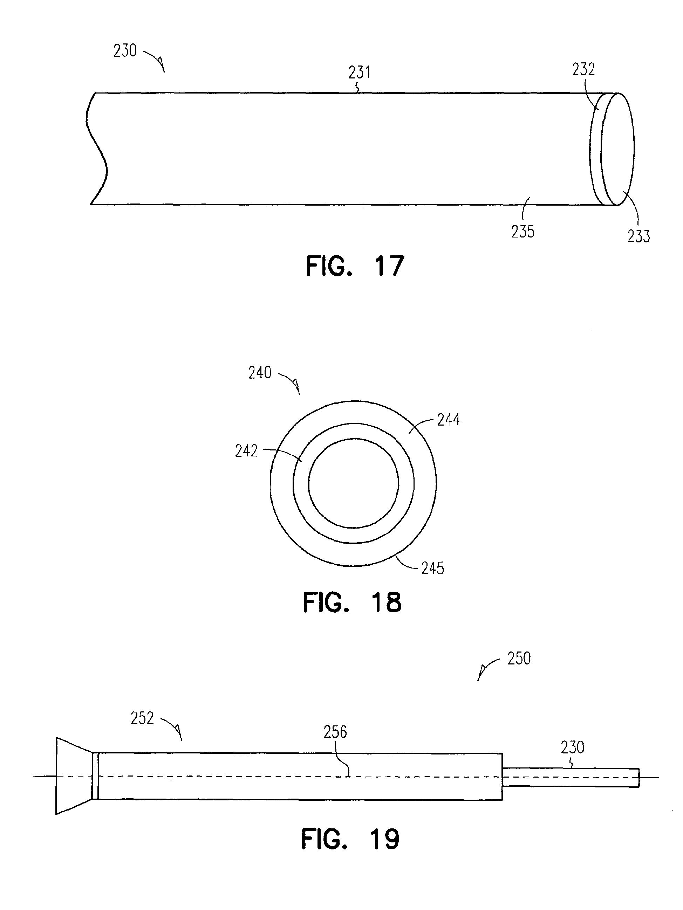

Embodiments of the invention described herein include device embodiments for sealing and repairing an aneurysm. One embodiment of the device includes a biocompatible polymeric hollow tube, such as is shown at 230 in FIG. 17, that is positionable within an aneurysm sac 24, shown in FIG. 3 and that functions to fill and to plug or seal the aneurysm The biocompatible polymeric hollow tube embodiment, illustrated at 230 in FIG. 17, includes a tubular main body 231 with an inner cylindrical surface 233 and an outer cylindrical surface 235, wherein some embodiments of the tubular main body 231 include a hydrogel and drugs and other agents incorporated into the hydrogel for healing the aneurysm. In another embodiment, the tubular main body 231 is coated with a coating 232 on either an inner cylindrical surface 233, an outer cylindrical surface 235 or both inner and outer cylindrical surfaces. The drugs and other agents are included as components of the coating 232.

For an embodiment illustrated in cross- section at 240 in FIG. 18, the tubular main body 231 includes an inner annular portion 242 made of a stiff hydrogel and an outer annular portion 244 made of a soft hydrogel foam. An encapsulation layer 245 covers the foam hydrogel. This layer is gelatin-like and comprises a water dissolvable polymer. The encapsulation layer, for some embodiments, has a time dependent rate of dissolution. The encapsulation layer is present to prevent premature swelling. The inner annular portion 242 may be stiffened as a consequence of an increased degree of cross-linkage as compared to the outer annular foam hydrogel 244, forming an outer jacket.

For other embodiments, the tubular main body is made of the soft hydrogel foam. The foam may have a range of cellular sizes. For some embodiments, the foam has a uniform cellular structure for the length of the tubular main body. For other embodiments, the foam cellular size is not uniform for the tubular main body. For some non-uniform embodiments, the foam cellular size is distributed in a non-random pattern and for other embodiments, the foam cellular sizing distribution is random. For additional embodiments, the tubular main body is made of a stiff hydrogel.

For another embodiment, the tubular main body 231 is segmented with segments comprising foam and segments comprising a hydrogel or gelatin. The segmentation occurs along the length of the tubular main body.

Another embodiment of the device includes a tubular main body that is filled to make a biocompatible polymeric string, such as is shown schematically at 26 in FIG. 2, that is positionable within an aneurysm sac 24 and that functions to fill and to plug or seal the aneurysm. One biocompatible polymeric string embodiment comprises a hydrogel and drugs and other agents incorporated in the hydrogel for healing the aneurysm. A polymeric string embodiment, illustrated in cross-section at 50 in FIGs 2 and 3, includes a stiff hydrogel core 52 with a central portion 54, that in one embodiment includes a soft hydrogel foam portion that concentrically surrounds the core 52. A gel 56 provides a concentric outer coating or encapsulation of the soft hydrogel foam 54. String embodiments also include the horizontal and annular distribution of hydrogel, gelatin, and foam that have been described for hollow tubular main bodies. While a filled tubular main body is described as one string embodiment, it is understood that other string embodiments are suitable for use in the invention described herein.

As used herein, the terms "biocompatible polymeric string," "biocompatible polymeric hollow tube", and "biocompatible polymeric sleeve" are used to describe types of "detachable filler," and "aneurysm detachable filler." The term "biocompatible polymeric sleeve" is used interchangeably with "hollow tubular main body." The term "coil" as used herein, refers to a biocompatible polymeric hollow tube or a string having a coil configuration somewhere along the length of the hollow tube or string. It is understood that reference to "biocompatible polymeric sleeve" or "string" also includes embodiments having at least one coil portion.

The biocompatible polymeric hollow tube embodiments 230 and 240 and string embodiments 26 include, in some embodiments, a radiopaque marker such as barium sulfate, tantalum, gold, tungsten or platinum, bismuth oxide, bismuth subcarbonate, and the like. The use of the marker enables a physician to determine proper placement and proper fill in the aneurysm sac 24.

Some embodiments of the biocompatible polymeric hollow tube 230 and central portion 54, of the biocompatible polymeric string, are made of a hydrogel foam portion which is swellable and which has a swell ratio of 10:1-2:1. For some embodiments, the biocompatible polymeric hollow tube or string is coated with a coating that includes materials such as growth factors, integrins, cell attachment proteins, cells, and genes and gene products to speed cell overgrowth. For other embodiments, the string or hollow polymeric tube is seeded with materials such as growth factors, integrins, cell attachment proteins, cells, and genes and gene products to speed cell overgrowth. These substances are added for speeding cell overgrowth and are, for other embodiments, added to other hydrogels. The tubular main body 231 for some embodiments, and foam portion 54 of a string for other embodiments, provide a desirable surface and surface area for rapid cell ingrowth. The hydrogel foam or other similar material is shapable at the aneurysm neck to form a smooth, closed surface at the aneurysm neck.

Swellable materials for use in the invention embodiments described herein include acrylic based materials. For one string embodiment, the core material is stiffer than the outer material, as shown in FIG. 2. In particular, FIG. 2 shows a cross-sectional area of a material 50 with the core hydrogel 52 and the surrounding foam hydrogel 54. An encapsulation layer 56 covers the foam hydrogel. This layer is gelatin-like and comprises a water dissolvable polymer. The layer 56, for some embodiments, has a time dependent rate of dissolution. The encapsulation layer is present to prevent premature swelling. The internal core hydrogel 52 may be stiffened as a consequence of an increased degree of cross-linkage as compared to the outer foam hydrogel 54, forming an outer jacket. In another embodiment, the core of the hydrogel string is a soft core metal wire.

The biocompatible hollow sleeve of the invention is formed to make a long continuous, hollow cylinder. The cylinder is placed into an aneurysm in a continuous fashion until angiographic filling is achieved. The cylinder material is then cut or detached. A wire is positioned within the sleeve to add support to the sleeve and to aid in moving the sleeve to an aneurysm site.

For the string embodiment, the biocompatible polymeric material is fabricated to form a long, continuous cylinder with a core surrounded by a jacket of soft, swellable hydrogel coated with a water soluble material, such as gelatin or other substance to prevent premature swelling.

The long continuous hollow cylinder or string is placed into an aneurysm in a continuous fashion until angiographic filling is achieved. The hollow cylinder or string material is then cut or detached. The encapsulation layer of the sleeve embodiment and string embodiment dissolves and allows the outer jacket material to swell to much greater filling volumes than are possible with platinum coils.

While a hydrogel is described, it is understood that other biocompatible, swellable materials are suitable for use in the hollow sleeve and string embodiments of the present invention. Other materials include cellulose acetate, ethylene vinyl alcohol copolymers, polyethylene, polypropylene, polyurethane, polyacrylonitrile, polyvinylacetate, cellulose acetate butyrate, nitrocellulose, copolymers of urethane/carbonate, copolymers of styrene/maleic acid, or mixtures thereof. A hydrogel/polyurethane foam is also usable in the hollow tube or string of the invention described herein.

In one embodiment, the detachable filler material includes a hydrophilic polyurethane. Other materials that are usable for either coatings for the aneurysm filler materials or the filler materials themselves include acrylamides such as hydroxypropyl methacrylamide, which is a hydrogel; isopropyl acrylamide, a thermoresponsive material; ethyl acrylamide, pH responsive material; and dicarboxymethylaminopropyl methacrylamide, which is a hydrogel. Other filler materials include methacrylates such as dimethyl amino ethyl methacrylate; oligo-dimethacrylate n-butyl acrylate, shape memory plastic; and hydroxyethyl methacrylate, which is a hydrogel. Other filler materials include cyclodextrans, synthetic elastin polymers such as protein gels, poly chelating and amphiphilic polymers.

Other hydrogel materials suitable for use in the aneurysm filler invention described herein include n-vinyl pyrrolidone, acrylic acid, sodium acrylate, acrylamido methyl propanesulfonic acid, sulfopropyl acrylate potassium salts, acryloyoxy ethyltrimethyl-ammonium methyl sulfate, albumin and gelatin modified by sulfate and poly (met acrylic acid) poly isopropyl acrylamide. Detachable filler materials also include hyaluronic acid conjugates, polyanhydrides, glycolipids, polysaccharides, and halamines, silicone, polysulfone, polyamide, polyimide, polyester, polytetrafluoroethylene, polyvinyl chloride, epoxy, phenolic, neoprene, polyisoprene, and a combination thereof. For some embodiments, the aneurysm string or sleeve 211 includes a polymer that has a melt temperature of between 200 to 600°F. For some embodiments, the string or sleeve 211 includes a conductive polymer that has a melt temperature of 200 to 600°F.

Thermoresponsive polymers are polymers that swell upon reaching body temperature but do not swell by hydration. pH sensitive polymers swell upon reaching physiologic pH values. Shape memory polymers are polymers which can be given a shape outside the body. Shape memory polymers return to an original shape with either hydration, thermal or pH changes. Each of these types of swellable polymers, those swellable by hydration, those swellable by heat and those swellable by pH are suitable for use in embodiments of the invention described herein.

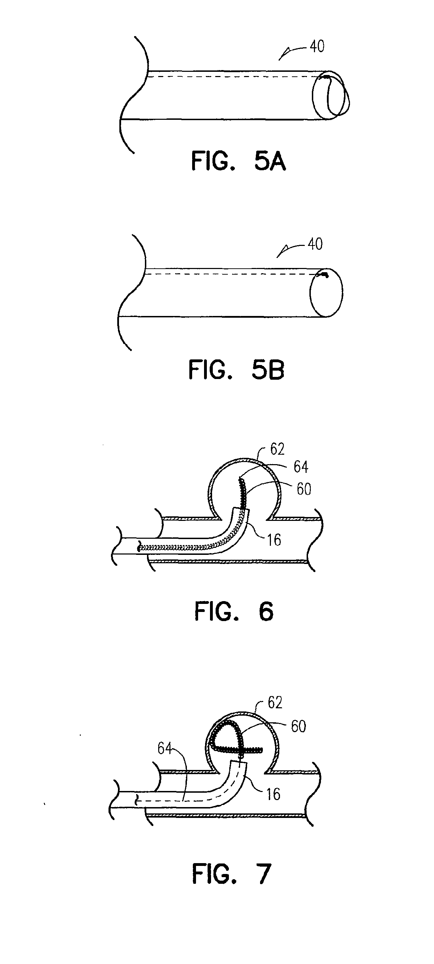

Another embodiment of the aneurysm filler of the invention includes a polymer-based, coil-like structure that is fabricated with soft biocompatible polymers such as PTFE, urethanes, polyolefins, nylons and so forth, such as is shown at 60 in FIGs. 6 and 7. Coil embodiments include hollow coils such as 60 and filled coils. The sleeve, coil and string embodiments are fabricated by direct forming, machining, laser cutting, injection molding or coiling/braiding.

These string, and hollow tubular main body structures also include embodiments fabricated with materials that include biodegradable materials such as PLA, PGA, PLGA, polyanhydrides and other similar biodegradable materials. The bio-active compound is selected from a group that includes an antithrombotic agent, an antiplatelet agent, an antimitotic agent, an antioxidant, an antimetabolite agent, an anti- inflammatory agent, and a combination thereof. A use of biodegradable materials provokes a wound healing response and concomitantly eliminates a mass effect of the filled aneurysm over time. For some embodiments, the biodegradable materials are seeded with materials such as growth factors, fibronectin, heparin, derivations of fibronectin, peptide mimics of fibronectin, laminin, vitronectin, thrombospondin, gelatin, collagen and subtypes thereof, gelatin, polylysine, polyornithine, and other adhesive molecules or derivatives or mimics of other adhesive molecules, integrins, cell attachment proteins, cells, and genes and gene products to speed cell overgrowth.

The aneurysm filler material described herein may be one or more of polymeric and polymeric hybrids such as PEBAX, Grilamids, polyester, and silica. Materials also include reabsorbables such as PGLA, PEG, PGLA and base polymer. Materials further include textiles such as rayon, nylon, silk, Kyeon, Kevlar, and cotton. Materials also include biopolymers such as collagen, filaments, and coated polymeric material. Materials further include elastomers such as urethanes, silicones, nitriles, Teco Flux, carbothane, and silicone hybrids.

The textile materials may be knits or woven and may be expandable. The textiles include polybutester such as Novatyil, PGA (Dexon), PLA (polylactic acid), polyglactin acid (Vicryl), polydiaxanone (POS) and polylyconate (Maxon).

Pusher materials for the proximal shaft include Grilamids, nylon (12 30% glass (PARG)), polyamide, filled HDPE, polybutylene terephthalate, rigid polyurethane and polypropylene, that is 30% glass filled.

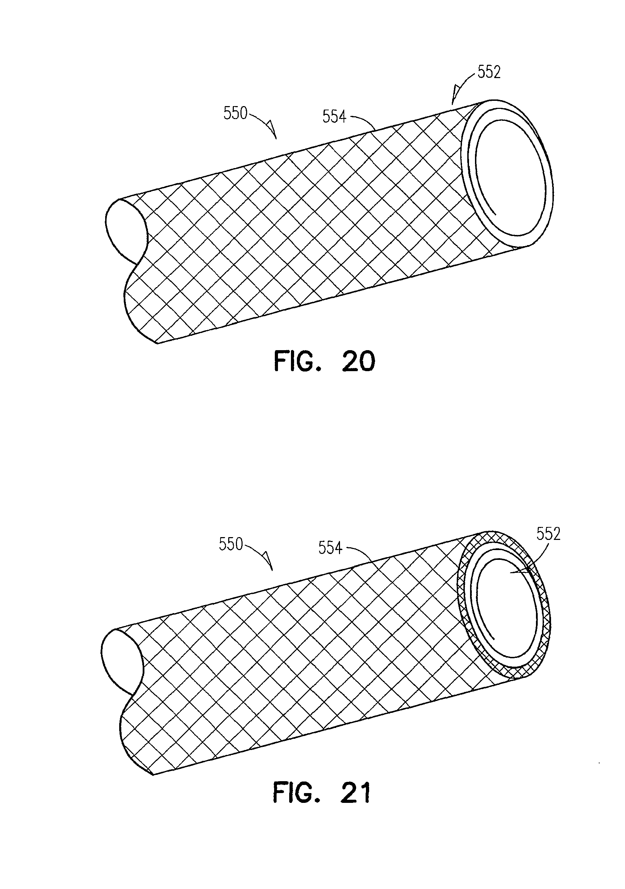

One embodiment of a filler that includes fabric is illustrated at 550 in FIGs. 20 and 21. The filler 550 is solid for some embodiments and hollow for other embodiments. The filler shown in 550 includes a polymeric main body 552 and an outer layer 554 that includes a woven fabric. For some embodiments, the woven fabric is rolled. The outer fabric layer 554 is adhered to the polymeric main body 552. The woven fabric or textile material includes, for some embodiments, one or more of polyester, nylon, absorbable material and fabric such as silk, suture material, and filter fabric.

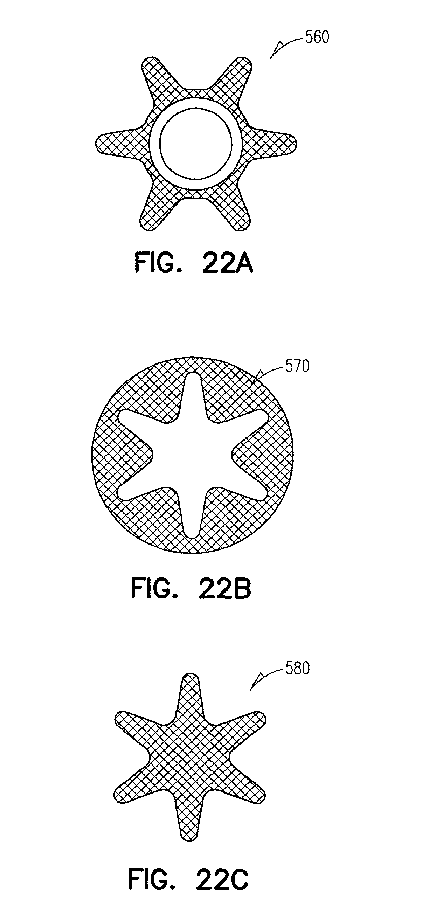

The fabric or textile or both may be adhered to the polymeric main body to form a variety of cross-sectional symmetries, some of which are shown at FIGs. 22A, 22B, and 22C. FIG. 22A illustrates a polymeric or metallic inner annular main body 560 defining a hollow core for some embodiments and a solid core for other embodiments, Fabric 562 is positioned about the annular main body to form a "star-like" shape.

A second embodiment, shown at 22B, shows a solid core 570 having a "star-like" shape and a fabric adhered to the solid core 570 to form an annulus. A third embodiment, shown at 580 in FIG. 22C, shows a "star-shaped" filler. The filler in the embodiment 580 is solid.

For some embodiments, lubricious materials such as hydrophilic materials may be used to coat the aneurysm filler. One or more bioactive materials may also be included in the composition of the core. The term "bioactive" refers to any agent that exhibits *effects in vivo,* for example, a thrombotic agent, a therapeutic agent, and the like. Examples of bioactive materials include cytokines; extra-cellular matrix molecules (e.g., collagen); trace metals (e.g., copper); matrix metalloproteinase inhibitors; and other molecules that stabilize thrombus formation or inhibit clot lysis (e.g., proteins or functional fragments of proteins, including but not limited to Factor XIII, α2-antiplasmin, plasminogen activator inhibitor-1 (PAI- 1) or the like)). Examples of cytokines that may be used alone or in combination in practicing the invention described herein include basic fibroblast growth factor (bFGF), platelet derived growth factor (pDGF), vascular endothelial growth factor (VEGF), transforming growth factor beta (TGF-β), and the like. Cytokines, extra-cellular matrix molecules, and thrombus stabilizing molecules are commercially available from several vendors such as Genzyme (Framingham, Mass.), Genentech (South San Francisco, Calif.), Amgen (Thousand Oaks, Calif.), R&D Systems, and Immunex (Seattle, Wash.). Additionally, bioactive polypeptides can be synthesized recombinantly as the sequence of many of these molecules are also available, for example, from the GenBank database. Thus, it is intended that embodiments of the invention include use of DNA or RNA encoding any of the bioactive molecules.

Furthermore, molecules having similar biological activity as wild-type or purified cytokines, matrix metalloproteinase inhibitors, extra-cellular matrix molecules, thrombus-stabilizing proteins such as recombinantly produced or mutants thereof, and nucleic acid encoding these molecules may also be used. The amount and concentration of the bioactive materials that may be included in the composition of the core member may vary, depending on the specific application, and can be determined by one skilled in the art. It will be understood that any combination of materials, concentration, or dosage can be used so long as it is not harmful to the subject.

Some embodiments of the string, or tubular main body filler have one or more emboli dissolving agents released locally to reduce the emboli. In other embodiments, the string, or tubular main body release oxygen and/or sugars to nourish the patient's brain cells. In other embodiments, the string, or tubular main body releases vasodilators such as nitrous oxide or heparin to increase the available oxygen transport. In other embodiments, the string, or tubular main body releases growth factors, which may improve healing or create new vessels.

For some embodiments, the string 12 in FIG. 3 and 60, in FIG. 6 or tubular main body 230 or 240 is coated with one or more of collagen, fibrin, or other bioactive materials, that have been described herein, for rapid healing. It is understood that other materials, such as those described above, that aid in rapid healing are suitable for use in a coating. The coating is applied for some embodiments, by deposition and for other embodiments, by application. For other embodiments, the tubular main body 230 and string 12 are made entirely of collagen.

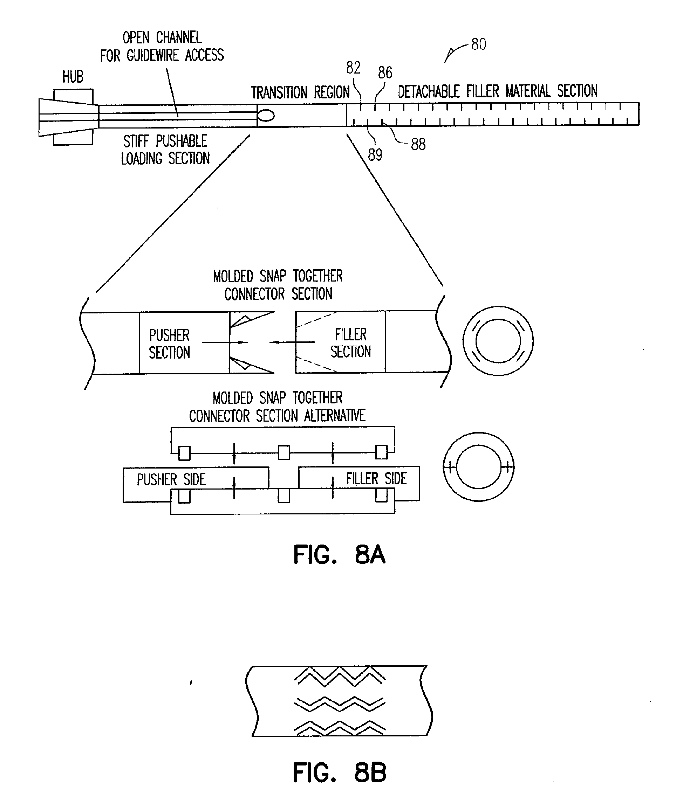

Some string, and hollow tubular main body embodiments include cuts or slits such as are shown at 82, 84, 86 and 88 as shown in FIGs. 8A and 8B. The cuts or slits mechanically increase the range of motion of some embodiments of the string, shown at 80. The cuts or slits also impart to the hollow sleeve, or string 80, a greater range of motion, flexibility, and more conformation embodiments. For other embodiments, the string, or tubular main body's flexibility is increased by cutting out semi-circles and other cutout shapes in the string. For some embodiments, the cut-outs or slits are randomly distributed and for other embodiments, the cut-outs or slits are ordered along the length of the hollow sleeve or string. One hollow sleeve embodiment includes cut-outs as shown in FIG. 8B. For some embodiments, the cut-outs or slits are positioned in order to increase the flex of the string or hollow sleeve at specific locations along the length of the string or hollow sleeve.

For some embodiments, the hollow tubular main body or string is made of one or more polymers with a flex modulus within a range of 5 ksi to 200 ksi (kilopounds per square inch).

### Solid Filler

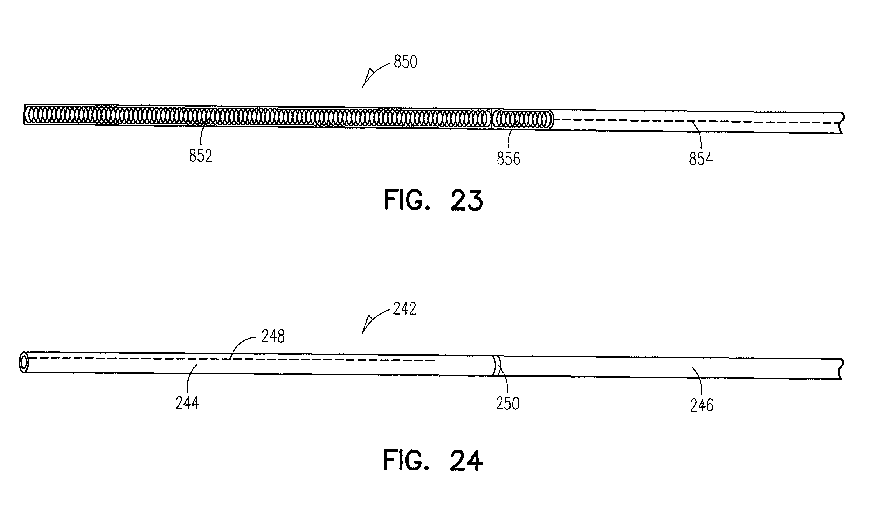

One filler embodiment, shown at 850 in FIG. 23 includes a solid filler 852, a pusher 854 and an insert joint 856 positioned between the solid filler 852 and the pusher 854. In one embodiment, a saline/contrast injection is used to detach the solid filler 852 from the pusher 854. The solid filler 852 includes a solid string that includes material that is inserted into the pusher 854. The pusher 854 has a high column strength, imparted by, in some embodiments, PEEK. For some embodiments, the insert joint 856 attaches to the filler and the pusher in a pressure fit.

The solid filler 852 has a greater tungsten, or similar material, loading capacity without greatly impacting the integrity of the material. The solid filler 852 also has greater strength during delivery.

To detach the filler, saline or contrast is used to pressurize the inside of the pusher and "inject" the filler into the aneurysm. The pusher 854 includes a distal end that is used to insert any filler left behind in the microcatheter. The filler may also be shipped in different lengths to accommodate different sized aneurysms.

### Ribbon Filler

Another filler embodiment, shown at 142 in FIG. 24, includes a filler 144 and a pusher 146. For some embodiments, the filler 142 is made from a tube having a perforation 148. The perforation is strong enough to allow the tube to be filled with a filler. Upon filling, the tube weakens and breaks at the perforation and forms a ribbon. The perforation 148 does not extend to a weld 150 that attaches the filler 144 to the pusher 146. Thus, the weld 150 forms a strong bond between the filler 144 and the pusher 146. The ribbon filler 144 has a thin, flat, ribbon-like shape that enables greater filling of the filler into an aneurysm.

### Braided Filler

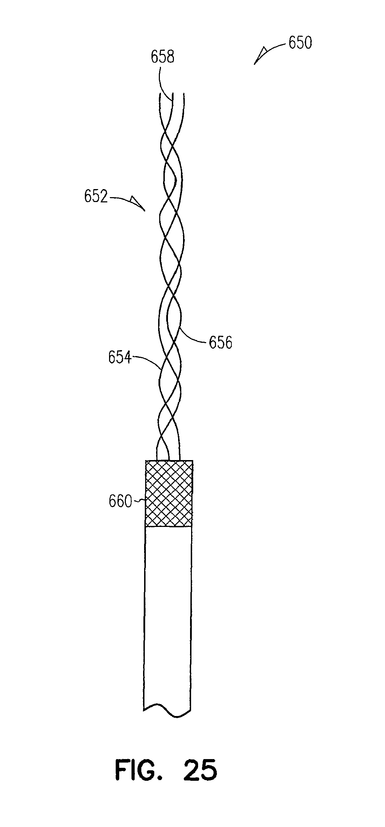

One other filler embodiment, illustrated at 650 in FIG. 25, includes a solid braided filler main body 652 that includes multiple strands of cable 654, 656, and 658 that are twisted together to make the cable filler main body 652. While three strands of cable are shown in FIG. 25, it is understood that the braided main body may include two or more strands of cable. The filler embodiment, 650 also includes a pusher 660 and a weld that attaches the multiple strands of cable to the pusher 660. The strands of cable 654, 656, and 658 are polymer based and may be loaded with radiopaque material. For some embodiments, the strands are welded at periodic junctions to hold the strands together. The strands, for some embodiments, may be melt separated using an external heat source. Some strand embodiments are coated with a slippery coating. Some embodiments are coated with a bioactive coating. The strands may be varying durometer measurements axially. Strands may be of different durometers. For some embodiments, a strand may have different durometers. For some embodiments, a distal section of selective strands is removed to make the distal end of the braided filler main body 652 more flexible.

The biocompatible polymeric sleeve, or string 26 is deployed to an aneurysm sac 24 through a lumen of a micro catheter, such as is shown at 250 in FIG. 19, which is disposed within the aneurysm sac 24. The microcatheter lumen 250 is positioned within a lumen 12 of an implant catheter, such as is illustrated at 10 in FIG. 1. The hollow tube for some embodiments and string for other embodiments is guided at 21 of the implant catheter.

In one other embodiment, a core wire 256 includes soft noble metal, gold, platinum, silver, or other noble metallic material. The core wire 256 is positioned within the annulus of the hollow tube 230 for some embodiments and is used instead of the stiff polymer core 52 to make the string for other embodiments. Suitable metals and alloys for the wire making up the coil include the Platinum Group metals, especially platinum, rhodium, palladium, rhenium, as well as tungsten, gold, silver, tantalum, and alloys of these metals. These metals have significant radiopacity and their alloys may be tailored to accomplish an appropriate blend of flexibility and stiffness. The materials are also largely biologically inert. Additional coating materials, such as polymer, or biodegradable materials, as discussed previously, may be added to the surface of the core member to improve the lubricity, healing properties, or thrombogenic properties of the vaso-occlusive detachable string, coil or sleeve.

The core wire 256 may also be any of a wide variety of stainless steels if some sacrifice of radiopacity may be tolerated. Suitable materials of construction, from a mechanical point of view, are materials that maintain their shape despite being subjected to high stress. Certain "super-elastic alloys" include nickel/titanium alloys, copper/zinc alloys, or nickel/aluminum alloys.

Titanium/nickel alloys known as "nitinol" may also be used in core wire embodiments. These are super-elastic and very sturdy alloys that will tolerate significant flexing without deformation even when used as a very small diameter wire. If nitinol is used in the core wire, the diameter of the core wire may be significantly smaller than that of a core member that uses the relatively more ductile platinum or platinum/tungsten alloy as the material of construction.

The core wire 256 may also be made, in some embodiment, of radiolucent fibers or polymers (or metallic threads coated with radiolucent or radiopaque fibers) such as Dacron (polyester), polyglycolic acid, polylactic acid, fluoropolymers (polytetrafluoroethylene), Nylon (polyamide), or silk.

In another embodiment, illustrated in FIGs. 6 and 7, a hollow sleeve, or string, is transported to an aneurysm sac with the microcatheter. The hollow sleeve is delivered into an aneurysm sac 62 over a wire 64 which is positioned within the aneurysm sac. The hollow tube or string is delivered over the wire 64 or 256 and is positioned within the aneurysm sac 62 with or without requiring the microcatheter to enter the aneurysm.

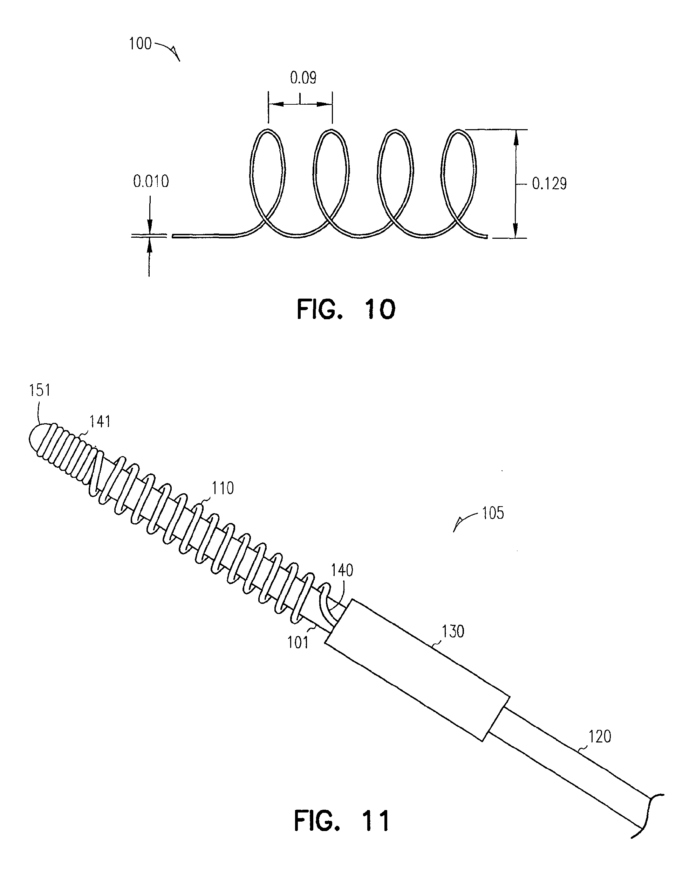

For some embodiments, at least a portion of the string or hollow sleeve is formed into coil loops, such as shown at 100 in FIG. 10. To make the loops of the coils, string or hollow sleeve material was extruded. Coils were formed, in one embodiment, using a guidewire with a Teflon coating, which acted as a shape forming wire. The string or hollow sleeve material was positioned around the shape forming wire. For one embodiment, shown in FIG. 10, four complete loops were formed. In general, the polymer was heated to its glass transition temperature and then quenched. After the ice water quench, the polymer was removed from the shape forming wire for a hollow tube. The coils were measured to determine the recovery diameter and the effects of different doses of cross-linking radiation. For some embodiments, particularly embodiments that include a polyolefin, further cross-linking was performed on the coil-shaped string or hollow tube by subjecting the coiled hollow tube or string to E-beam, or cross-linking radiation at dosages within a range of 2.5 mrad and 20 mrad. In another embodiment, coils were formed by cross-linking alone.

In another embodiment, a pre-formed coil loop is inserted into an end of the string or sleeve and is permanently attached in place. The coil loops may be formed of polymers of the same type as the string or sleeve or may be fabricated of wire such as platinum in the form of GDC coils of 2D or 3D shape.

The coil loops 100 formed at a distal end of a hollow tube require straightening prior to insertion into a microcatheter, for insertion into a patient. The coil loops are straightened in one embodiment by insertion of a wire into the tube lumen, or, with an outer straightening sheath over the outside of the tube. When the straightened coil loops exit the microcatheter, the loops return to their coiled loop shape.

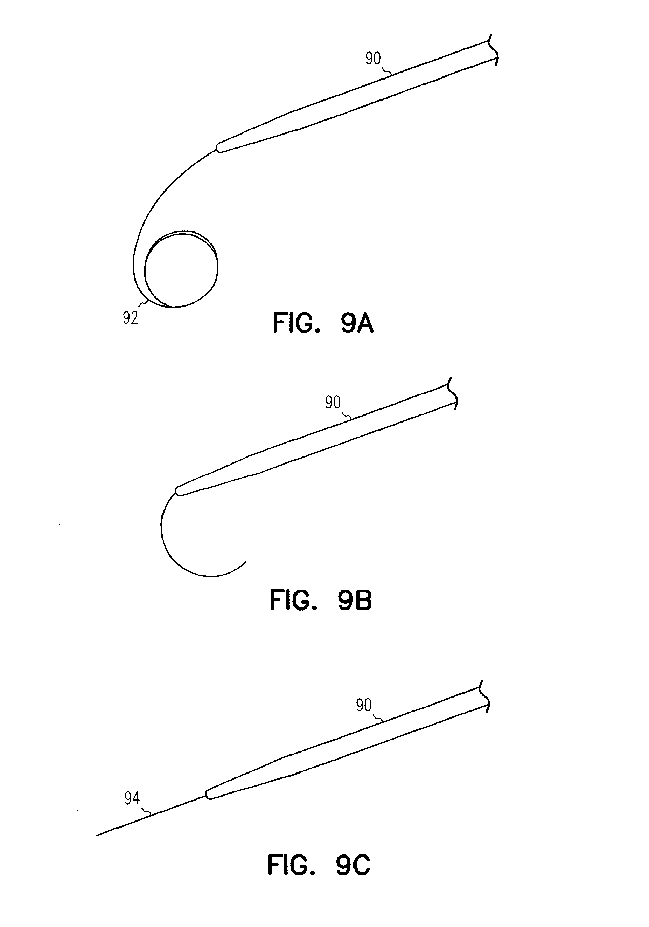

One embodiment of the implant catheter assembly of the invention includes a coil loop straightening sheath as illustrated at 90 in FIGs. 9A, 9B and 9C. The coiled loop straightening sheath straightens a coil loop 92 for insertion into a catheter. A fully straightened loop is shown at 94 in FIG. 9C. For other embodiments, a wire is inserted into the tube lumen. The straightening sheath is positioned over the outside of the string or sleeve or coil and is used to straighten the coil loops. With this embodiment, the tube itself is retained in a single position, avoiding the added friction to the wire in the lumen of the string or sleeve.

The sleeve, coil 60 or string is insertable into the aneurysm either at the microcatheter tip or outside the microcatheter in small pushable sections. These embodiments do not require the catheter tip to enter the aneurysm, although the tip may enter the aneurysm. The wire gains access to the aneurysm sac and also functions as a rail to guide the polymer coil 60 or hollow string into the aneurysm. The wire 64 imparts strength and support sufficient to permit the coil or string to be pushed into the aneurysm without the material itself being required to have that support "built-in."

In one embodiment, the wire includes a detacher wire portion, which allows for separation of the fill material from fill material remaining within the microcatheter. For some embodiments, the fill material is detachable anywhere along the length of the string or hollow tube. For some embodiments, the filler material is detached more than one time. The coil tip includes a heater element and acts as a heater. The wire includes a heater element made of a material such as titanium, Nitinol, nickel chromium alloy, alloys of nickel and chromium, stainless steel, tungsten, iridium, niobium, copper, zinc and carbon fiber.

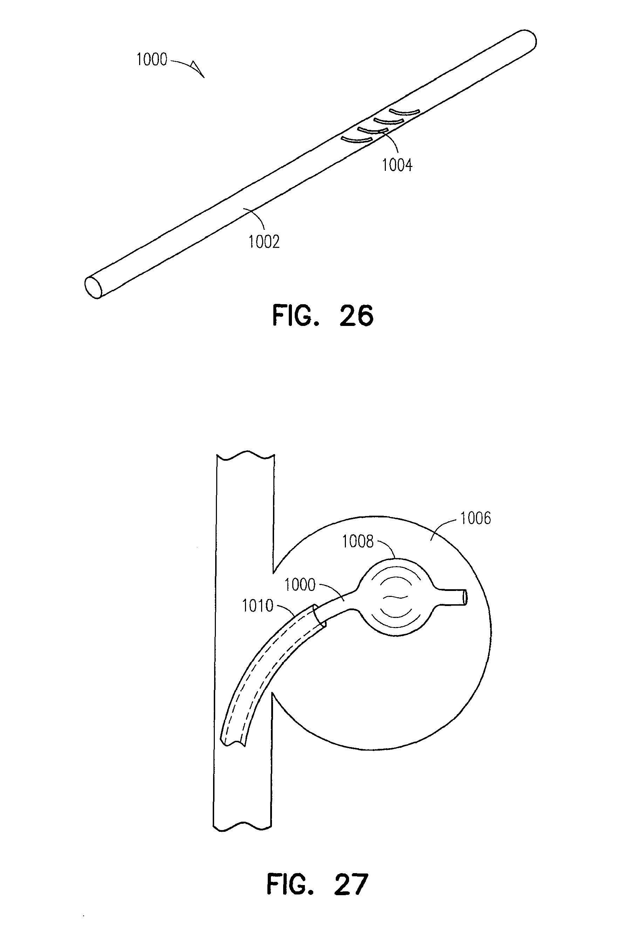

One embodiment of a distal tip is shown at 1000 in FIG. 26. The distal tip 1000 includes an elongate main body 1002 with a spiral slit 1004. The spiral slit 1004 is continuous for some embodiments and sequential in other embodiments.

The distal tip 1000 is shown and *in situ* expanded position in FIG. 27. When the distal top 1000 touches the wall of an aneurysm 1006, the tip fans and opens and initiates folding over to better collapse into the aneurysm 1006. A spiral 1008 realigns itself to return into a microcatheter 1010 over a wire.

The device includes a variety of heater sizes and shapes, as measured in diameters and lengths. The device is usable with direct DC power or via radio frequency, ultrasound, or other energy sources. The power requirements of the detacher device are variable and depend upon the tissue requirements and the size of the device.

Embodiments of the invention described herein also includes a method for sealing and repairing an aneurysm. The method comprises providing a biocompatible polymeric string, sleeve or coil as has been described herein. Also provided is an implant catheter, such as is shown at 10 in FIG. 1, that comprises a lumen 12 having a proximal end 14 and a distal end 16. The proximal end 14 comprises a manifold 18 with a port 20 for insertion of the biocompatible polymeric string or hollow tube. The biocompatible polymeric hollow sleeve or string is pushed through the lumen 12 to the distal end 16. The distal end 16, in one embodiment, terminates in a curved tip 22. The curved tip 22 is positionable within an aneurysm sac 24 as is shown in FIG. 2.

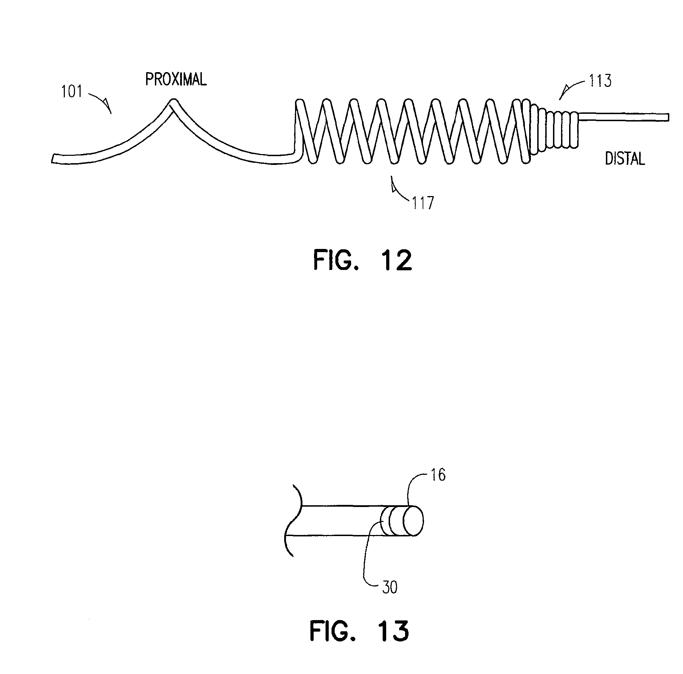

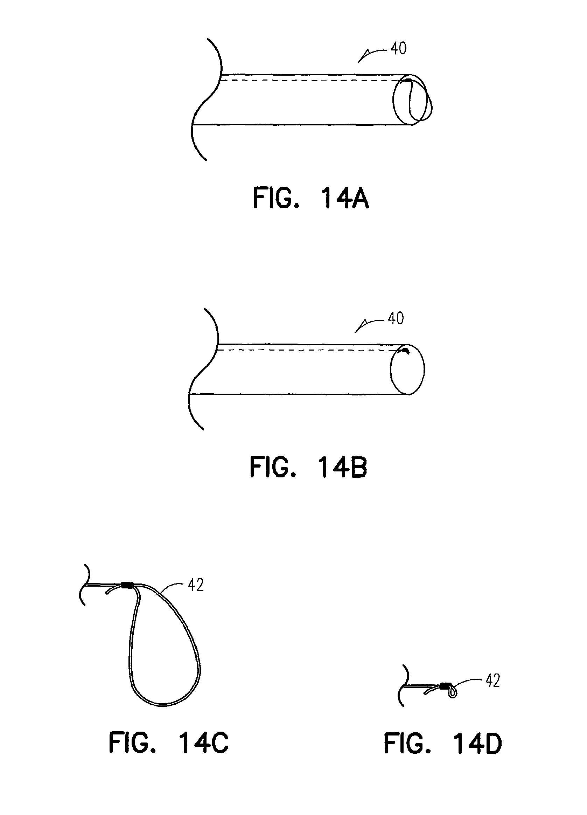

The biocompatible polymeric hollow sleeve or string may be detached in one embodiment, with a heater, such as is shown at 30 in FIG. 13 or cut with a mechanical cutter, shown at 40 in FIGs. 14A and 14B, located at the distal end 16 of the lumen of the microcatheter 250. In the embodiment in FIG. 13, the string 26 is detached with a heater which may be an electrical-based heater 30 or a laser.

In another embodiment illustrated at 40 in FIGs. 14A and 14B, the hydrogel string 26 or hollow sleeve is cut with a mechanical loop cutter 42. The loop cutter 42 may be manipulated in order to decrease the loop in diameter and cut through the polymer material 26.

The lumen 252 of microcatheter 250 in FIG. 19 has a generally circular cross-sectional configuration with an external diameter in a range of about 0.01 to 0.5 inches for cerebral vascular applications. The microcatheter 250 has an internal diameter ranging from 0.01 to 0.035 inches. The lumen 252 has sufficient structural integrity to permit the implant catheter 250 to be advanced to distal arterial locations without buckling or undesirable bending of the lumen 252.

Embodiments of the invention described herein also includes an aneurysm filler detacher wire assembly, one embodiment of which is illustrated at 105 in FIG. 11 and a method for making and a method for using the aneurysm filler detacher wire assembly 105. The method is usable in embodiments that include melt separation or polymeric softening separation or polymeric swelling or a combination of melt separation, polymeric softening of polymeric materials and polymeric swelling. For some embodiments, the heat generated by cutting stiffens an end of the filler material.

For one embodiment, the aneurysm filler includes a segment that includes a polymer having a melting point that is lower than the rest of the aneurysm filler. The segment is positioned to cover an area where the filler will be detached. The segment is large enough to increase flexibility of where the filler detachment occurs. In another embodiment, the segment includes materials that promote a sharp melting point.

In one embodiment, the detacher wire assembly 105 includes the following components: a wire assembly that includes a core wire 101, a heater element 110, a lead wire 120, a marker band 130, an electrical connection joint 141, a distal coil attachment 151, and an electrical connector 140.

The core wire 101, illustrated in FIG. 12, imparts structural integrity to the wire assembly and provides for axial push and pull force applied to wire assembly. The core wire 101 serves as conductor wire for an electrical circuit. The core wire increases in flexibility from a proximal end to a distal end.

The core wire 101 is made of one or more materials that include super elastic nitinol, medial grade stainless steels, MP35N, Beta Titanium. The material resistance of the core wire is 25 ohms to 250 ohms. One core wire embodiment is 35 to 120 ohms over 150 to 320 ohms.

In one embodiment, an element 110 acts as the heating element when current is applied to the detacher wire circuit. Heating acts to activate heating and/or swelling of the filler material. The heater element 110 is joined to the core wire 101 at 151. The heater element 110 is wound in separate segments of different diameters. The smaller "minor coil" 113 is designed for aposition to the core wire and provides for the distal laser joining. The larger "major coil" 117 serves as the heating element. The heater coil 110 is wound in separate segments of different diameters. The smaller minor coil 113 is designed for operation to the coil wire 101 and provides for the distal laser weld. The larger major coil 117 serves as the heating element. For some embodiments, the device includes only one heating element, rather than a "major coil" and a "minor coil."

The heater element 110 acts as the heating element when current is applied to the detacher wire circuit. The coil 151 is joined to the core wire 101 via laser welding, soldering, adhesive bonding, crimping, resistance welding. One embodiment employs laser welding.

The heater element 110 is made from materials that include nickel chromium alloys, shape memory nitinol, titanium, tungsten, iridium, niobium, copper, zinc and carbon-fiber, and stainless steel. Heater coil materials have a resistance that ranges from 5 ohms - 100 ohms over 0.5 - 15 cm. In one embodiment, materials have a resistance that ranges from 30 ohms - 60 ohms over 5 cm -10 cm.

The lead wire 120 provides a lead wire for the electrical circuit. The lead wire 120 is made from materials that include copper, super elastic Nitinol, Nickel, Aluminum, Platinum, Nichrome alloys. Material resistance ranges from 5 ohms - 100 ohms over 30 cm-350 cm. In one embodiment, resistance ranges from 30 ohms - 60 ohms over 5 to 10 cm. The heater element 110 is wound in separate segments of different diameters. The smaller minor element 113 is designed for operation to the coil wire 101 and provides for the distal laser weld. The larger major element 117 serves as the heating element.

The marker band 130 provides a radiopaque marker for imaging with a fluoroscope. The marker band 130 is made one or more of the materials described above for marker bands and helps the physician with positioning the wire in-situ for treatment. Specific marker band materials include Platinum, Tantalum, Gold, Tungsten, Platinum-Iridium. One embodiment includes materials of Platinum in a concentration of 80-90% and Iridium in a concentration of 10-20%.

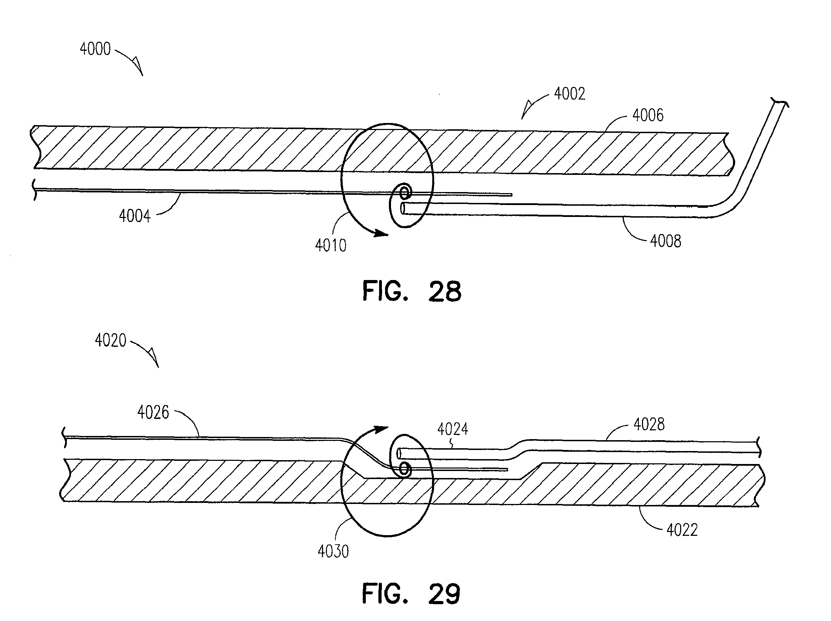

In another embodiment, illustrated at 4000 in FIG. 28, a main body 4002, includes a copper lead wire 4004, an insulated core wire 4006, and a coil 4008. A wrap 4010 that includes a radiopaque ribbon 4012 is wrapped around the copper lead wire 4004, the coil 4008 and the core wire 4006.

In one embodiment, the radiopaque ribbon 4012 includes a thin film made of materials that include one or more of platinum, tungsten, gold, tantalum and combinations of these materials. The radiopaque ribbon 4012 is wrapped around the copper lead wire 4004, coil 4008 and insulated core wire 4006. For some embodiments, the radiopaque ribbon 4012 is wrapped around the copper lead wire 4004 and the coil 4008. Next, the radiopaque ribbon 4012 is wrapped tightly around the insulated core wire 4006 several times to further bind the core 4006 to other components. For some embodiments, the radiopaque ribbon 4012 is sealed to the other components using an adhesive for some embodiments and soldering for other embodiments. For some embodiments, a combination of soldering and adhesive is employed.

In another embodiment shown at 4020 in FIG. 29, a core wire 4022 is ground to form a flattened area 4024. Within the flattened area, a copper wire 4026 and coil 4028 are bound by a radiopaque ribbon.

The electrical connection joint 141 joins the heater element 110 to the lead wire in FIG. 11. The electrical connection joint may be soldered, resistance welded, adhesive bonded, or crimped to the heater element 110 and the lead wire joint. In one embodiment, the electrical connector joint is soldered with silver solder.

The distal coil attachment occurs wherein the heater element 110 is attached to the core wire 101 at the distal tip. This attachment is accomplished by laser welding, resistance welding, soldering, adhesive bonding or crimping. In one embodiment, the attachment occurs by laser welding.

The electrical connector 141 joins the core wire 101 proximal end to a pin socket, and allows for connection of the wire assembly to a power supply for activation.

Electrical resistance of the assembled components of the detacher ranges from 75 ohms to 250 ohms. In one embodiment, the range is 300 cm length device of 120 to 200 ohms.

Current requirements for the detacher wire assembly range from 150 mA to 250 mA for some embodiments. The assembly operates within a range of 5 to 50 VDC. Current is supplied to the Detacher Wire assembly 105 through a direct current 2-50V power supply. A connector of the detacher wire is connected to an intermediary cable assembly that is then attached to power supply. As current is applied to the detacher wire assembly, the current flows through the lead wire 120 and Core Wire 101 terminating at the heater element 110. Due to its resistance the heater element begins to heat. The heat is then transferred to the surrounding implant polymer which causes, in some embodiments, melting, softening or swelling of the polymer resulting in polymer separation.

In one embodiment, the wire detacher operation is performed with the lumen of the implant catheter filled with a fluid, with the distal coil of the detacher wire encapsulated within a fluid bath within the polymer. The filler is softened, separated or swelled. The fluid can be tap water, deionized water, sterile water, Dextrose Solution 5% (D5W), or saline. If a conductive solution, just as saline for an example, is to be used as the bathing solution added electrical insulation is required on one or more of the core wire, heater coil, and/or solder joint to prevent current leakage and prevent rapid corrosive effects. This added electrical insulation can take the form of a deposition coating consisting of Parylene, Teflon, silica, ceramics, zirconia, titanium dioxide, alumina, and so forth.

The size range of the Detacher Wire 105 assembly is between 0.005" diameter and 0.035" diameter. For one embodiment, the size ranges from 0.008" diameter to 0.010" diameter to fit within a catheter with a lumen of 0.012" diameter to 0.014" diameter. The length of the heater coil is within 0.25 mm-5 mm. For one embodiment, the length of the wound major coil is 1.25 mm.

In one embodiment, an alumina deposition coating of 1-5 microns is placed upon the core wire, directly beneath the major coil.

The lead wire 120 is attached to the core wire 101 with an adhesive coating to bind the two together. This is accomplished via a method such as shrink tubing, dip coating, spray coating, deposition coating, or other conventional process.

In another embodiment, the detacher assembly includes a detachment feedback circuit. One aneurysm filler detachment feedback circuit described herein includes an electro-mechanical circuit for detecting when the aneurysm filler material has been separated. The device includes a feedback mechanism for alerting the operator that a separation has occurred.

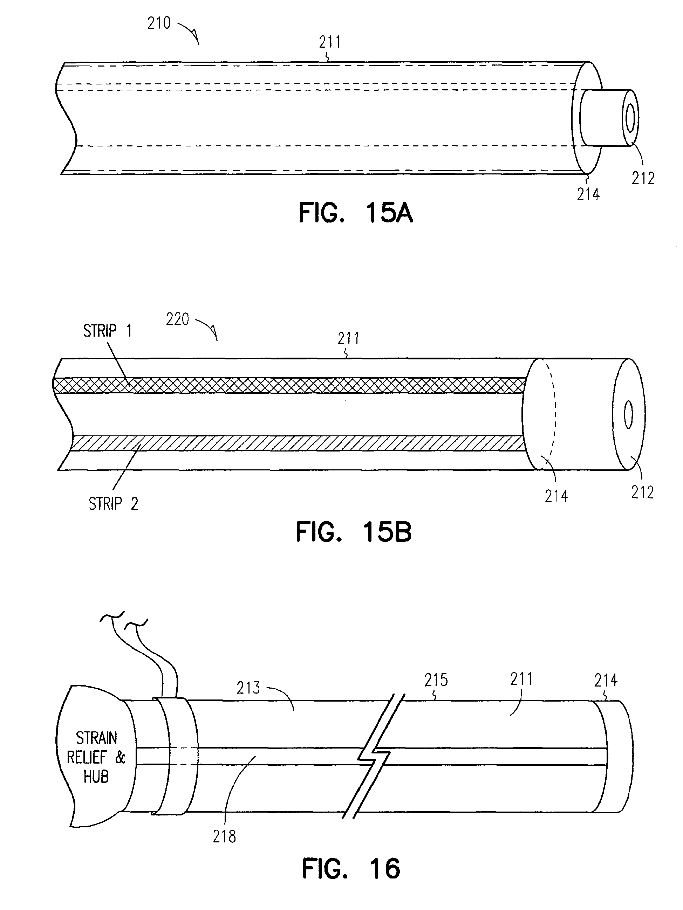

One embodiment of the feedback mechanism, illustrated generally at 210 in FIGs. 15A of the detacher assembly provides a positive electro-mechanical feedback mechanism to sense the separation or cutting of the aneurysm filler having a string, coil or sleeve conformation into two discrete lengths of the string, sleeve or coil. The feedback mechanism 210 includes an aneurysm filler having a string or sleeve conformation as shown at 211 in FIG. 16. When the aneurysm string 211 is cut, it forms two discrete lengths 213 and 215, shown in FIG. 16. In one embodiment, the aneurysm filler string or sleeve 211 includes one or more wire leads embedded into the annular wall of the string, annular hollow sleeve that are connected to a conductive ring 214 at one end of the cylinder, either at an inner annulus or an outer annulus, forming a circuit. In another embodiment, the aneurysm filler string or sleeve includes a deposit of a flexible conductive film, one deposit of which is shown at 218 on opposing surfaces of the string or film 211. Each of the films contacts the ring 214. In one other embodiment, a conductive layer is on an inside annular surface or an outside annular surface or both.

Conductive strips and ring usable in the feedback mechanism are made of one or more materials that include gold, platinum, silver, titanium, or tantalum. The conductive materials are corrosion resistant and coatings remain intact during the placement of the filler material. In one embodiment, the conductive materials are covered with a coating such as a hydrophilic coating to insulate the living being from the conductive materials. For some embodiments, growth factors such as those described herein overlay the conductive layer.

The conductive ring 214 at a proximal end of the aneurysm string or sleeve connects the conductive strips 218 or electrical wires to lead wires going to a power supply. In another embodiment, the lead wires connect the conductive strips 218 to a heat shrink/strain relief. In a third embodiment, the cylinder itself is conductive along its entire length. With this embodiment, a deposition coating forms a conductive ring on the outer diameter or inner diameter of the string or sleeve 211. In another embodiment, an embedded conductive ribbon is inserted in the wall of the aneurysm string or sleeve. The ribbon must be thin enough to break upon detachment. Thus, when the aneurysm filler string or sleeve is cut, the circuit is broken and activates an alarm system.

In embodiments 15A and 15B and 2, at the distal end of the aneurysm string or sleeve 211, the leads or conductive pathways 218 are connected to the conductive ring 214 which is also positioned in a portion of the wall of the string or sleeve 211 or is positioned on an inside or outside annular wall of the string or sleeve, as is shown in FIG. 21. In the third embodiment, the deposition coating forms a conductive ring on the outer diameter of the string or sleeve. On the proximal end the leads or conductive pathways are connected to an electrical circuit monitoring device that senses the resistance of the overall circuit in or on the catheter body by providing a low level power input through the closed loop circuit.

In another embodiment, at the proximal end, a conductive cylinder is connected to an electrical circuit monitoring device that senses the resistance of the conductive cylinder. With this embodiment, a calculation may be performed to determine the amount of filler added to an aneurysm. The resistance is proportional to the amount of filler added. The wires or flexible conductive coating extend from a hub, shown in FIG. 16, to a distal tip of the aneurysm filler string or sleeve in a way effective for creating an electrical circuit. The filler along with elements that conduct an electric current create a closed electrical circuit. When the filler is detached, the circuit is opened.

In one other embodiment, when the aneurysm filler string or sleeve is cut with heat, the electrical circuit is also cut, thus sending a change in resistance or change in the circuit integrity to the circuit-monitoring device which triggers an alarm indicating the change in the circuit. In this fashion, a user of the device will have a positive feedback of the separation of the material without having to visualize the separation or tactilely feel the separation occur.

One embodiment of the detachment mechanism also includes a feedback mechanism for detachment that provides information to a physician as to when the string is detached. In addition to providing information concerning whether the aneurysm filler material has been cut, the feedback mechanism may also provide information regarding the amount of filler added to the aneurysm, based upon the resistance measured prior to filler detachment. When current is applied, the resistance is measured. When the circuit is broken by cutting the aneurysm filler, the resistance is measured again. The change in resistance is associated with the length of the aneurysm filler added to the aneurysm.

In one other embodiment, when the aneurysm filler string or sleeve is cut with heat, the electrical circuit is also cut, thus sending a change in resistance or change in the circuit integrity to the circuit-monitoring device which triggers an alarm indicating the change in the circuit. In this fashion, a user of the device will have a positive feedback of the separation of the material without having to visualize the separation or tactilely feel the separation occur.

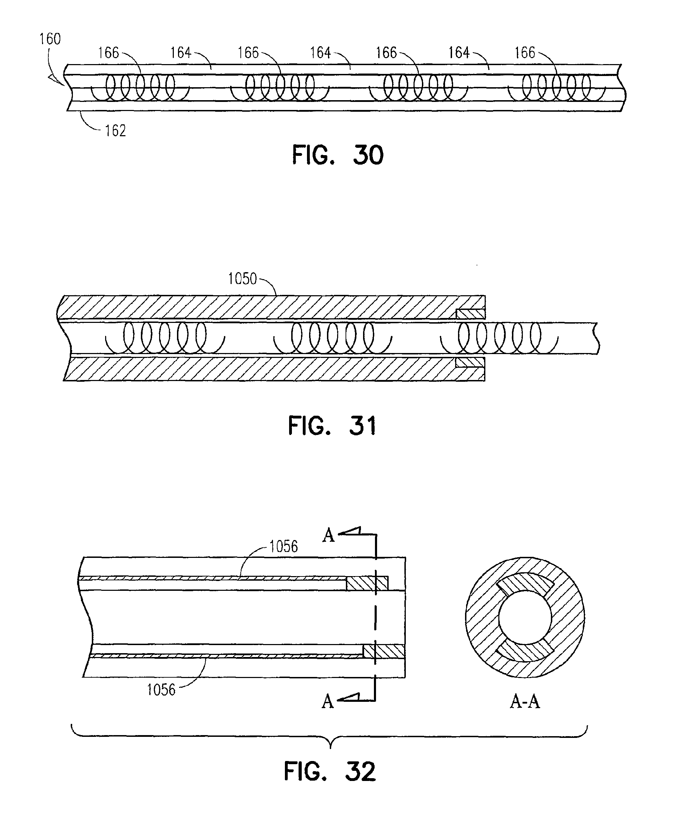

A system that combines an aneurysm filler 164, detacher 166, and delivery device 168 into one system is shown at 160 in FIG. 30. The system 160 works as an "all in one" type of device where multiple detachment coils, such as shown at 166 or other detachment mechanisms may be placed in the HF. The detachment mechanisms 166 detach a portion of the catheter from the system. Coils and other types of detachment mechanisms distal to the separated area are left behind in an aneurysm. The length of the material may of virtually any length.

The system 160 works, for some embodiments, in three ways. When component B is inserted into the aneurysm, component B serrates or cuts the connection so that all of the coils that were in the aneurysm would not be activated. The second way that component B functions is to run multiple copper lengths up to each coil and then push a button on the a detachment box to separate the coil. A third way is to have a set length of material and place the coil at a length in the material so that the same amount of material is detached every time.

One system embodiment, shown at 1050 in FIG. 31, includes a hollow stranded or beaded filler or multi-layered filler with detacher coils embedded at intervals along the way to be detached using an outer sheath or microcatheter containing tow leads on each side and a contact point at the distal end.

For some embodiments, lead wires, shown at 1056 in FIG. 32, are embedded or adhered to a wall of sheath connected to a conductor layer and bound at a distal tip which connects to a coil to close the electrical circuit for activation of a heater coil.

For some embodiments, the system includes a conductive layer or band that is not a continuous band. Rather, the band is of discrete length and both bands are offset to allow current to flow through the heater coil.

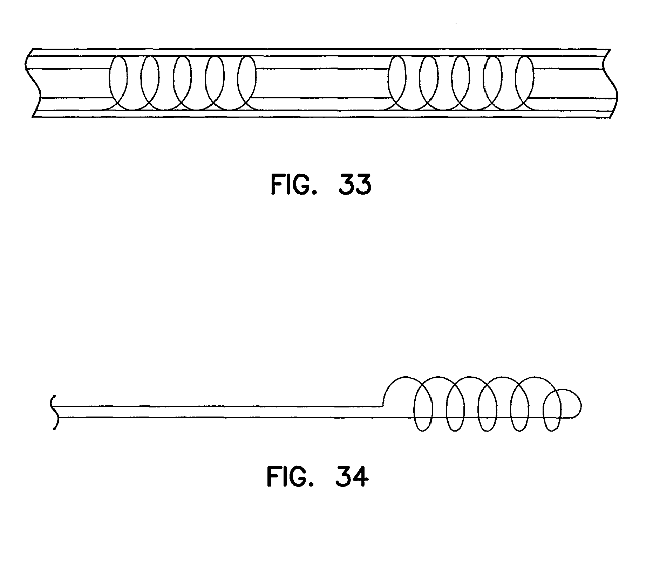

In another embodiment, coils embedded on an ID of filler detacher wire with two leads goes through as shown in FIG. 33 and FIG. 34. A line detacher coils up exactly inside a filler coil. Uninsulated sections of det. Coil make contact with filler coil redirecting current to the filler coil. Tension is applied to separate the filler proximal to the filler coil.

For some embodiments, the detacher wire assembly and optionally the feedback mechanism are used to cauterize or ablate tissue. The cauterization or ablation is performed *in situ.*

For some embodiments, the detacher wire assembly and optionally the feedback mechanism are used to cauterize or ablate tissue. The cauterization or ablation is performed *in situ.*

Certain embodiments are provided by one or more of the following paragraphs.

Paragraph 1. A method for treating an aneurysm, comprising:
providing a biocompatible polymeric sleeve, string or coil or combination of sleeve, string or coil, made from a material selected from one or more of a group consisting of an acrylamide, a methacrylate, cyclodextran, synthetic elastin polymer, poly chelating amphiphilic polymers, hydrogels, hyaluronic acid conjugates, polyanhydrides, glycolipids, polysaccharides, and halamines, natural hydrogel, a synthetic hydrogel, silicone, polyurethane, polysulfone, cellulose, polyethylene, polypropylene, polyamide, polyimide, polyester, polytetrafluoroethylene, polyvinyl chloride, epoxy, phenolic, neoprene, polyisoprene, and a combination thereof.;
transporting the sleeve, string or coil to an aneurysm;
filling the aneurysm with the sleeve, coil, or string; and
detaching the sleeve, string or coil.

Paragraph 2. The method of paragraph 1 wherein the sleeve, string or coil is delivered to an aneurysm over a wire.

Paragraph 3. The method of paragraph 2 wherein the wire comprises a detacher portion that detaches a segment of the sleeve, coil or string that treats the aneurysm.

Paragraph 4. The method of paragraph 3 wherein the detacher portion comprises a heater element.

Paragraph 5. The method of paragraph 1 wherein the string is detached with a loop cutter.

Paragraph 6. The method of paragraph 1 wherein the string is detached with a laser cutter.

It will be understood that the embodiments of the present invention which have been described as illustrative of some of the applications of the principles of the present invention. Various modifications may be made by those skilled in the art without departing from the spirit and scope of the invention.

## Claims

1. A kit for treating an aneurysm, comprising:
a biocompatible polymeric sleeve, string or coil made from a material selected from one or more of an acrylamide, a methacrylate, cyclodexdran, synthetic elastin polymer, poly chelating amphiphilic polymer, hydrogel, hyaluronic acid conjugate, natural hydrogel, a synthetic hydrogel, silicone, polyurethane, polysulfone, cellulose, polyethylene, polypropylene, polyamide, polyimide, polyester, polytetrafluoroethylene, polyvinyl chloride, epoxy, phenolic, neoprene, polyisoprene, one or more of a thermoresponsive polymer, a pH sensitive polymer, or a shape memory polymer and a combination;
a catheter for transporting the string to an aneurysm site; and
a mechanism for detaching the string.

2. The kit of claim 1 wherein the string comprises a stiff hydrogel core or a hydrogel foam.

3. A biocompatible sleeve, or string, comprising:
a hollow, annular main body comprising foam contacting the annular main body.

4. The biocompatible string sleeve or coil of claim 3 wherein the annular main body comprises a hydrogel.

5. A catheter from 0.005 to 0.035 inches in cross-section, comprising: a core wire; a heater element positioned over the core wire; a lead wire; a closed circuit for generating heat in the heater coil; and a dielectric coating.

6. A method for notifying a user when a biocompatible sleeve, string, or coil has been cut, comprising:
providing the sleeve, string, or coil comprising a cylindrical main body and a conductive element attached to the cylindrical main body, the string, sleeve or coil having a conformation effective for forming an electrical circuit; and
interfacing the sleeve, string or coil with a mechanism that signals to the user that the string, sleeve or coil is cut when an electrical circuit is broken.

7. A method for providing information regarding quantity of filler used to fill an aneurysm when the filler comprises a biocompatible sleeve, string, or coil has been detached, the method comprising:
providing the sleeve, string, or coil comprising a cylindrical main body and a conductive element attached to the cylindrical main body, the string, sleeve or coil having a conformation effective for forming an electrical circuit;
interfacing the sleeve, string or coil with a mechanism that signals to the user that the string, sleeve or coil is cut when an electrical circuit is broken; and
determining the amount of the sleeve, string or coil added to the aneurysm.

8. An aneurysm filler comprising a filler, a pusher and a joint attaching the filler to the pusher.

9. The aneurysm filler of claim 8, wherein the pusher comprises one or more of Grilamids, nylon (12 30% glass (PARG)), polyamide, filled HDPE, polybutylene terephthalate, rigid polyurethane and polypropylene, that is 30% glass filled.

10. The aneurysm filler of claim 8, wherein the filler comprises at least one of:
a) a main body comprising a woven or textile material, optionally forming an outer surface of the main body.
b) a main body which is star-shaped.

11. The aneurysm filler of claim 8 wherein the filler comprises a solid string and optionally further comprises two or more strings that are braided.

12. The aneurysm filler of claim 8, wherein the filler comprises a ribbon filler.

13. The aneurysm filler of claim 12, wherein the ribbon filler comprises a hollow tube having a perforation which was breached when the hollow tube was filled with a filler.

14. An aneurysm filler comprising one or more strands having a length and shaped adapted to fit within an aneurysm.
